# EUROPEAN PATENT SPECIFICATION

(11) **EP 1 922 977 B1**
(45) Date of publication and mention of the grant of the patent: **06.11.2013**
(21) Application number: 06783229.5
(22) Date of filing: 08.09.2006
(51) Int. Cl.: A61B 1/00, A61B 5/07

(54) **IMAGE DISPLAY DEVICE**
BILDANZEIGEEINRICHTUNG
DISPOSITIF D'AFFICHAGE D'IMAGE

(30) Priority: 09.09.2005 JP 2005263087
(43) Date of publication of application: 21.05.2008
(73) Proprietor: OLYMPUS MEDICAL SYSTEMS CORP., Tokyo 151-0072 (JP)
(72) Inventor: KIMOTO, Seiichiro c/o Olympus Medical Systems Corp., Tokyo 151-0072 (JP)
(74) Representative: von Hellfeld, Axel
(86) International application number: PCT/JP2006/317869
(87) International publication number: WO 2007/029819

(56) References cited:
- EP-A1- 1 790 277
- EP-A1- 1 918 870
- EP-A1- 1 952 751
- EP-A2- 1 288 839
- EP-A2- 1 316 911
- WO-A1-2004/103167
- WO-A1-2006/022269
- JP-A- 2001 028 047
- JP-A- 2004 070 942
- JP-A- 2004 216 008
- JP-A- 2004 334 789
- JP-A- 2005 131 031
- US-A1- 2004 249 291
- US-A1- 2004 267 122
- US-A1- 2008 172 255

## Description

### TECHNICAL FIELD

The present invention relates to an image display apparatus for displaying a series of intra-subject images captured along a time-series.

### BACKGROUND ART

In recent years, a swallowable capsule endoscope having an imaging function and a radio communication function has been proposed in a field of endoscopes, and an intra-subject information acquiring system for acquiring intra-subject images by using the capsule endoscope has been developed. The capsule endoscope travels through inside a body cavity, for example, through inside organs such as the stomach and the small intestine, during a period from when the capsule endoscope is inserted from a mouth of a subject for an observation (examination) until when the capsule endoscope is naturally excreted, while following peristaltic motion of the organs. The capsule endoscope functions to obtain an intra-subject image for, for example, every 0.5 seconds.

While the capsule endoscope travels inside the subject, the images captured by the capsule endoscope are sequentially transmitted to a receiving device placed outside of the subject via a radio communication. The receiving device having a radio communication function and a memory function stores images received from the capsule endoscope inside the subject in a memory sequentially. The subject carrying such a portable receiving device can freely move during the period from when the capsule endoscope is inserted until when the capsule endoscope is naturally excreted. After the capsule endoscope is naturally excreted from the subject, a doctor or a nurse can make an image display apparatus import the images stored in the memory of the receiving device, and display the images of organs of the subject to make a diagnosis on the subject (see Patent Document 1, for example).

The image display apparatus has a report creating function for creating a report (medical records) in which the diagnosis result and the like of the subject are described. Specifically, the image display apparatus enters patient information such as a patient name and the like, the diagnosis result, images of the subject captured by the capsule endoscope, and the like in a predetermined format to create the report for the subject. In this case, with respect to the images of the subject to be entered in the report, the image display apparatus can enable appending a comment such as a finding of the images input by the doctor, the nurse, or the like by using a keyboard or the like.

Patent Document 1: Japanese Patent Application Laid-Open No. 2003-19111

Document US 2004249291 A1 discloses an apparatus according to the preamble of claim 1.

### DISCLOSURE OF INVENTION

### PROBLEM TO BE SOLVED BY THE INVENTION

However, in the conventional image display apparatus described above, in the case of appending a comment to a plurality of images entered in the report for the subject, it is necessary to input the comment for each of the images even though there are images to which the same comment is to be appended among the plurality of images. Therefore, there are many cases where an input operation of repeatedly inputting the same comment to the plurality of images is required, resulting in a problem of causing a cumbersome input operation for creating the report for the subject.

In view of the foregoing, an object of the present invention is to provide an image display apparatus capable of simplifying an input operation for creating a report for a subject.

### MEANS FOR SOLVING PROBLEM

An image display apparatus according to one aspect of the present invention is defined by independent claim 1. Specific embodiments of the image display apparatus are defined by the dependent claims.

In the image display apparatus according to the present invention, the group processor may change an arrangement order of at least two of the groups arranged in a predetermined order.

### EFFECT OF THE INVENTION

According to the present invention, even when there are images to which the same comment is to be appended among the plurality of images, it is possible to input the comment to the every group in which at least two images to be appended with the same comment are grouped, instead of repeatedly inputting the same finding to the plurality of images. Thus, according to the present invention, it is advantageous that the image display apparatus in which the input operation for preparing the report for the subject can be simplified.

### BRIEF DESCRIPTION OF DRAWINGS

FIG. 1 is a schematic diagram exemplifying a configuration of an intra-subject information acquiring system including an image display apparatus according to an embodiment of the present invention;
FIG. 2 is a schematic block diagram exemplifying a configuration of the image display apparatus according to the embodiment of the present invention;
FIG. 3 is a schematic diagram of one example of a display screen of the display unit;
FIG. 4 is a schematic diagram exemplifying a state where a window for setting an output mode of an image file is displayed;
FIG. 5 is a schematic diagram for explaining an operation of creating a normal moving image file;
FIG. 6 is a schematic diagram for explaining an operation of creating a combined moving image file;
FIG. 7 is a schematic diagram for explaining an operation of creating a cut-out moving image file;
FIG. 8 is a schematic diagram of one example of a window displayed on the display unit for performing an output operation and the like of an image file;
FIG. 9 is a schematic diagram of one example of a window displayed on the display unit for preparing a report for a subject;
FIG. 10 is a schematic diagram of one example of a window for separating thumbnails displayed in a thumbnail display area into desired groups;
FIG. 11 is a schematic diagram exemplifying a window showing a state where grouped-images are set by the group processor;
FIG. 12 is a schematic diagram of one example of a report prepared by the report preparing unit; and
FIG. 13 is a schematic diagram for explaining a data lock process of an original file performed by the lock processor.

### EXPLANATIONS OF LETTERS OR NUMERALS

1 SUBJECT
2 CAPSULE ENDOSCOPE
3 RECEIVING DEVICE
3a to 3h RECEIVING ANTENNAS
4 IMAGE DISPLAY APPARATUS
5 PORTABLE RECORDING MEDIUM
11 INPUT UNIT
12 DISPLAY UNIT
13 CARD I/F
14 INFORMATION INPUT/OUTPUT I/F
15 STORAGE UNIT
15a EXAMINATION FOLDER
15b MANAGEMENT FOLDER
15c INPUT/OUTPUT FOLDER
16 CONTROL UNIT
16a DISPLAY CONTROLLER
16b IMAGE FILE CREATOR
16c REPORT PREPARING UNIT
16d GROUP PROCESSOR
16e IMAGE EXTRACTING UNIT
16f OUTPUT PROCESSOR
16g LOCK PROCESSOR
16h INPUT PROCESSOR
50 MAIN-IMAGE DISPLAY AREA
60 FILE DISPLAY AREA
70 DISPLAY OPERATION ICON GROUP
80 SUBIMAGE DISPLAY AREA
81 SCROLL BAR
91 IMAGE OUTPUT ICON
92 REPORT ICON
93 to 95 ICONS FOR SETTING THE NUMBER OF DISPLAY IMAGES
96 EXIT ICON
101 STILL IMAGE FILE DISPLAY AREA
102 MOVING IMAGE FILE DISPLAY AREA
103 OUTPUT FILE DISPLAY AREA
104 FILE DESIGNATION ICON
105 REMOVE ICON
110 DISPLAY OPERATION ICON GROUP
120 OUTPUT DESTINATION SELECTING AREA
130 OK ICON
201 THUMBNAIL DISPLAY AREA
202 SCROLL BAR
203 GROUPED-IMAGE DISPLAY AREA
204 SCROLL BAR
210 NORMAL-IMAGE DISPLAY AREA
211 to 213 MARKER ICONS
214 COMMENT PREPARING AREA
215 DICTIONARY AREA
216 ENTRY AREA
221 UNDO ICON
222 GROUPING ICON
223 GROUP ARRANGEMENT ICON
224 REPORT PREPARATION ICON
225 CLOSE ICON
250 GROUPING AREA
251 SCROLL BAR
261 OK ICON
262 CANCEL ICON
301 PATIENT INFORMATION
302 EXAMINATION INFORMATION
303 DIAGNOSIS INFORMATION
304 EXAMINATION RESULT INFORMATION
400 EXTERNAL COMPUTER
401 PORTABLE RECORDING MEDIUM
A1 to A5, D1, D2, B1 to B5, C1 to C5, E1 to E5 IMAGES
CPG PARTIAL IMAGE GROUP
CF COPY FILE
F1 to F4 EXAMINATION FILES
Gr1 to Gr4 GROUPED-IMAGES
K CURSOR
M1, M2 NORMAL MOVING IMAGES
MA COMBINED MOVING IMAGE
MB CUT-OUT MOVING IMAGE
OR ORIGINAL FILE
P1 IMAGE
PG IMAGE GROUP
PL1 to PL4 IMAGING REGION MARKERS
PV PREVIEW AREA
RM1 to RM5 REPORT MARKERS
SP1 to SP5 THUMBNAILS
S SLIDER
TS TIME SCALE
W1 to W6 WINDOWS

### BEST MODE(S) FOR CARRYING OUT THE INVENTION

Exemplary embodiments of an image display apparatus according to the present invention will be explained in detail with reference to the accompanying drawings. It should be noted that the present invention is not limited to the embodiment.

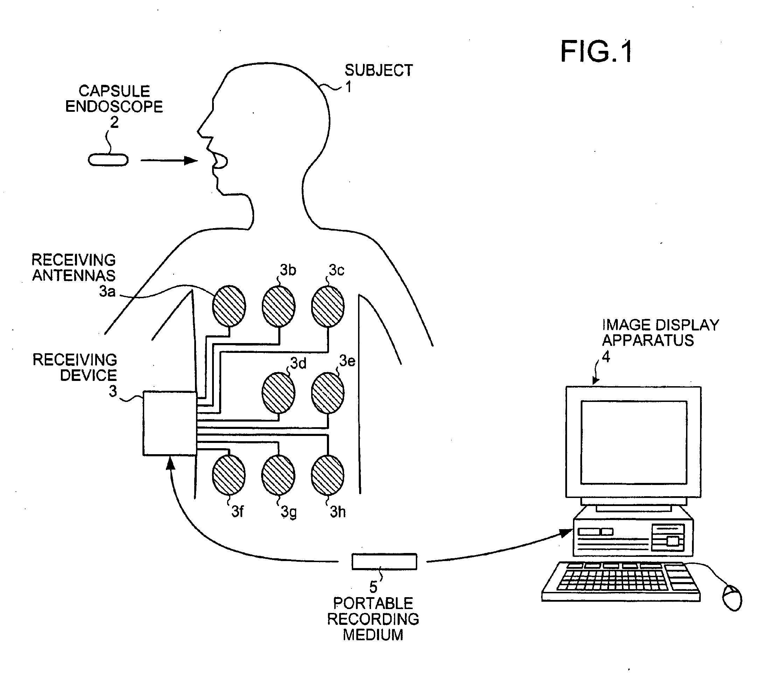

FIG. 1 is a schematic diagram exemplifying a configuration of an intra-subject information acquiring system including an image display apparatus according to an embodiment of the present invention. As shown in FIG. 1, the intra-subject information acquiring system includes a capsule endoscope 2 which travels along a path inside a subject 1 to capture an image of the inside of the subject 1; a receiving device 3 which receives a radio signal transmitted from the capsule endoscope 2 to store an image contained in the radio signal; an image display apparatus 4 which displays the image stored in the receiving device 3, i.e., the image captured by the capsule endoscope 2; and a portable recording medium 5 which transfers information between the receiving device 3 and the image display apparatus 4.

The capsule endoscope 2 has an imaging function of sequentially capturing images of the inside of the subject 1 along a time-series, and a radio communication function of transmitting a radio signal containing the captured images to the outside. The capsule endoscope 2 swallowed by the subject 1 passes through the esophagus, and travels inside the body cavity of the subject 1 in accordance with the peristalsis of the lumen of the alimentary canal. While traveling, the capsule endoscope 2 captures images of the inside of the subject 1 at a predetermined interval, for example, for every 0.5 seconds and transmits the images of the inside of the subject 1 to the receiving device 3 via a predetermined radio wave, sequentially.

The receiving device 3 is, for example, connected to a plurality of receiving antennas 3a to 3h which are dispersed and arranged on the outside surface of the subject 1, receives the radio signal from the capsule endoscope via any one of the plurality of receiving antennas 3a to 3h, and acquires images of the inside of the subject 1 based on the received radio signal. The receiving device 3, to which the portable recording medium 5 is detachably attached, stores the images acquired based on the radio signal from the capsule endoscope 2, i.e., the images captured by the capsule endoscope 2, sequentially. Thus, the receiving device 3 stores an image group of the inside of the subject 1 captured by the capsule endoscope 2 along a time-series in the portable recording medium 5.

The receiving antennas 3a to 3h, which are realized by using a loop antenna for example, receives the radio signal transmitted from the capsule endoscope 2. The antennas 3a to 3h are, as shown in FIG. 1, dispersed and arranged at predetermined positions on the outside surface of the subject 1 respectively, for example, at positions corresponding to the path of the capsule endoscope 2 inside the subject 1. The receiving antennas 3a to 3h may be dispersed and arranged at predetermined positions on a jacket worn by the subject 1. In this case, with the jacket worn by the subject 1, the receiving antennas 3a to 3h are arranged at predetermined positions on the outside surface of the subject 1 corresponding to the path of the capsule endoscope 2 inside the subject 1. It is only necessary to arrange at least one receiving antenna with respect to the subject 1, and the number of the antenna is not specially limited to eight.

The portable recording medium 5 is a recording medium capable of being carried such as the compact flash (registered trademark) and the like. The portable recording medium 5 can be detachable with respect to the receiving device 3 and the image display apparatus 4, and has a configuration capable of outputting and recording information when attached to the both. Specifically, when attached to the receiving device 3, the portable recording medium 5 sequentially stores various types of information such as images captured by the capsule endoscope 2 and acquired by the receiving device 3, and the respective imaging times thereof. In contrast, when attached to the image display apparatus 4, the portable recording medium 5 outputs the stored information such as the images captured by the capsule endoscope 2 as described above to the image display apparatus 4. In this manner, the information stored in the portable recording medium 5 is imported to the image display apparatus 4. In addition, information of the subject 1 in a capsule endoscope examination is written in the portable recording medium 5 in the image display apparatus 4. The capsule endoscope examination means the insertion of the capsule endoscope 2 into the inside of the subject 1, and the observation of the images captured by the capsule endoscope 2.

The image display apparatus 4 is for displaying images and the like captured by the capsule endoscope 2. Specifically the image display apparatus 4 has a configuration like a work station in which various types of information stored by the receiving device 3 into the portable recording medium 5 is imported to acquire various types of information such as the images and the like captured by the capsule endoscope 2, and the images of the inside of the subject 1 are displayed based on the acquired information. The image display apparatus 4 has an image display function for displaying images in series contained in the image group of the inside of the subject 1, and further has a process function for allowing a user such doctor or a nurse to observe (examine) the images of the inside of the subject 1 and make a diagnosis on the subject 1. In this case, the user can display the images of the inside of the subject 1 sequentially on the image display apparatus 4, observe (examine) regions of the inside of the subject 1, for example, the esophagus, stomach, small intestine, large intestine, and the like, and make a diagnosis on the subject 1 based on the observation.

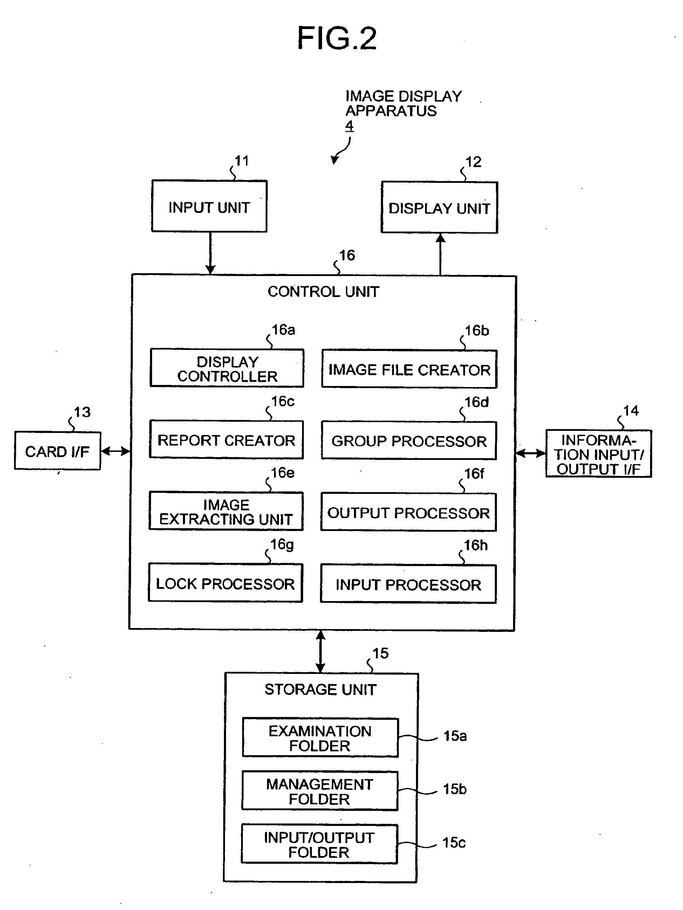

Next, a configuration of the image display apparatus 4 will be explained. FIG. 2 is a schematic block diagram exemplifying the configuration of the image display apparatus 4 according to the embodiment of the present invention. As shown in FIG. 2, the image display apparatus 4 includes an input unit 11 which inputs various types of information for the observation of the images of the inside of the subject 1; a display unit 12 which displays various types of information such as the images and the like of the subject 1 for making an examination and a diagnosis on the subject 1 on a screen; and a card interface (I/F) 13 which imports information such as the images of the inside of the subject 1 stored in the portable recording medium 5. The image display apparatus 4 further includes an information input/output I/F 14 which inputs/outputs various types of information such as the images of the subject 1 with an external computer, for example; a storage unit 15 which stores various types of information such as the images and the like of the subject 1; and a control unit 16 which controls the driving of each component of the image display apparatus 4.

The input unit 11, which is realized by using a keyboard, a mouse, and the like, inputs instruction information for instructing the control unit 16, and patient information of the subject 1 to the control unit 16 via an input operation by the user. The patient information is registered in the receiving device 3 by way of the portable recording medium 5 for example, so as to perform an initial setting of the receiving device 3 as a receiving device for performing the capsule endoscope examination on the subject 1. Such patient information includes, for example, patient name, sex, birth date, patient ID, and the like of the subject 1.

The display unit 12, which is realized by using various types of display such as a CRT display, a liquid crystal display, or the like, displays various types of information which the control unit 16 instructs to display. In this case, the display unit 12 displays various types of information such as the images and the like of the subject 1 captured by the capsule endoscope 2 for making an observation and a diagnosis on the subject 1. Specific example of a display screen of the display unit 12 will be described later.

The card I/F 13 is for importing the information stored in the portable recording medium 5. Specifically, the card I/F 13, to which the portable recording medium 5 is detachably attached, reads out the information stored in the portable recording medium 5, and transmits the acquired storage information to the control unit 16. The card I/F 13 writes information which the control unit 16 instructs to write, for example, the patient information and the like in the attached portable recording medium 5.

The information input/output I/F 14 is for performing an input/output of various types of information between the image display apparatus 4 and an external computer or peripheral equipment. Specifically the information input/output I/F 14 can detachably attach a portable recording medium such as a flexible disk (FD), a compact disk (CD), a digital versatile disk (DVD), and the like, and is realized by using a drive and the like which performs a reading process or a writing process of various types of information with respect to the attached portable recording medium. The information input/output I/F 14 has a configuration capable of connecting peripheral equipment such as a printer via a predetermined cable. The information input/output I/F 14 writes the information which the control unit 16 instructs to write in the portable recording medium of the drive, or outputs the information which the control unit 16 instructs to output to the peripheral equipment such as the printer. The information input/output I/F 14 reads out the information which the control unit 16 instructs to read from the portable recording medium of the drive, and transmits the acquired information to the control unit 16.

The storage unit 15, which is realized by using an information recorder which is capable of storing and reading information in a RAM, an EEPROM, a hard disk, or the like, stores information which the control unit 16 instructs to write, and transmits the storage information which the control unit 16 instructs to read to the control unit 16. The storage unit 15 has an examination folder 15a which retains and manages an examination file of the subject including the image group of the subject, patient information, the examination information (examination date and examination ID, for example), and the like; a management folder 15b which retains and manages various kinds of files such as medical records (report) of the subject prepared by a process function of the image display apparatus 4, still images, moving images, and the like; and an input/output folder 15c which retains and manages various kinds of files input/output in communication with the outside.

As described above, the control unit 16 controls the driving of each component of the image display apparatus 4, for example, the input unit 11, the display unit 12, the card I/F 13, the information input/output I/F 14 and the storage unit 15, and controls the input/output of information between the components. The control unit 16 creates an examination file, and stores the examination file in the examination folder 15a. In the examination file, the image group of the subject 1 acquired by way of the portable recording medium 5, time information related to the imaging time of each image contained in the image group, the patient information of the subject 1, and the examination information of the subject 1 are associated together as a file. In this case, the control unit 16 retains and manages each examination file stored in the examination folder 15a for every subject or examination ID, for example.

The control unit 16 includes a display controller 16a which controls a display operation of the display unit 12 with respect to the various types of information; an image file creator 16b which creates a desired image file of a still image or a moving image based on the image group contained in the examination file; a report preparing unit 16c which prepares a report in which the diagnoses result and the like of the subject are described; and a group processor 16d which separates the images entered in the report prepared by the report preparing unit 16c into desired groups. The control unit 16 further includes an image extracting unit 16e which extracts an image file or an image necessary for creating the report from the image group contained in the examination file; an output processor 16f which performs an output process of data such as an image file and a report file to be output to the outside; a lock processor 16g which bans updating of the original file whose copy file is output to the outside; and an input processor 16h which performs an input process of data such as an image file and a report file to be input from the outside.

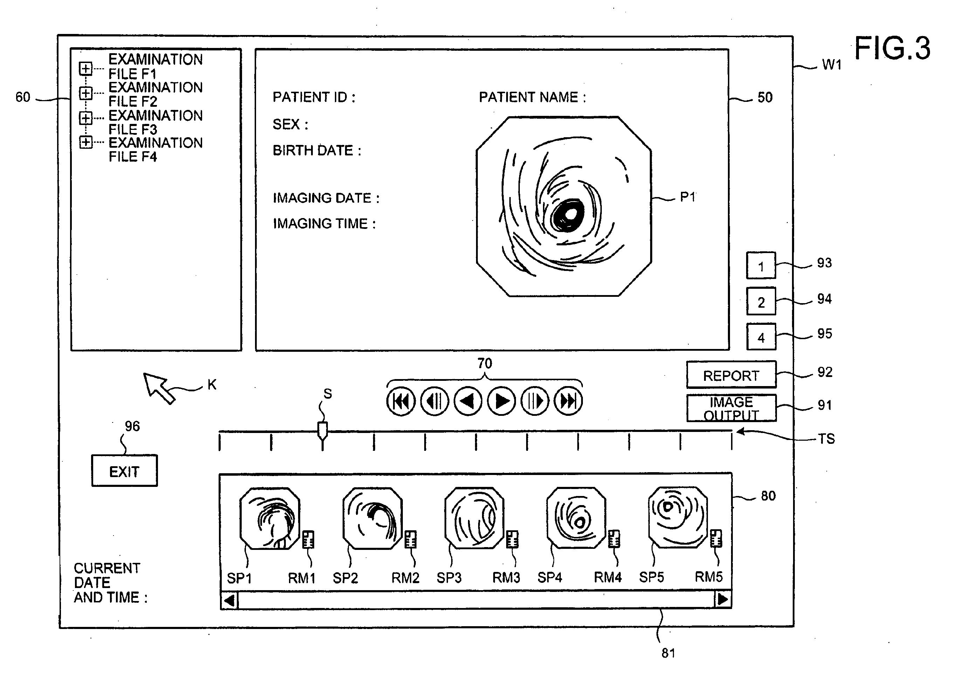

Next, a display screen of the display unit 12 will be exemplified and an operation of the display controller 16a for controlling the display operation of the display unit 12 will be explained. FIG. 3 is a schematic diagram of one example of the display screen of the display unit 12. When the control unit 16 performs a predetermined log-in process, the display controller 16a displays a window W1 as shown in FIG. 3 on the display unit 12.

As shown in FIG. 3, the window W1 has a main-image display area 50 which displays images captured by the capsule endoscope 2; a file display area 60 which displays a list of examination files stored in the examination folder 15a of the storage unit 15; a display operation icon group 70 for performing an operation of displaying each image of the image group contained in the examination file in the main-image display area 50; a subimage display area 80 which displays a thumbnail corresponding to an image selected among images sequentially displayed in the main-image display area 50; and a scroll bar 81 for performing a scroll operation of the thumbnail displayed in the subimage display area 80.

The window W1 has a time scale TS which indicates an elapsed time since the start of the imaging of each image contained in the examination file selected from the file display area 60; and a slider S which indicates an elapsed time of the image displayed in the main-image display area 50 during the elapsed time indicated by the time scale TS. The window W1 further has an image output icon 91 for outputting an image file created by the image file creator 16b; a report icon 92 for creating a report of a subject; icons for setting the number of display images 93 to 95 for setting the number of images to be displayed in the main-image display area 50 at a time; and an exit icon for closing the window W1.

Else, a cursor K for performing a designation of the examination file, a selection of an image, an operation of various kinds of icons and the like via an input operation of the input unit 11, and information of current date and time are present in the window W1.

The display controller 16a controls to display the image group contained in the examination file designated via an input operation of the input unit 11 among the list of examination files F1 to F4 displayed in the file display area 60, for example. Specifically, the user uses the input unit 11 to perform an input operation for designating a desired examination file among the examination files F1 to F4 with the cursor K pointed to the desired examination file. The display controller 16a controls to display an image among the image group contained in the designated examination file as an image P1 in the main-image display area 50 in series. In this case, the display controller 16a, for example, controls to display the image P1 in series along a forward direction or a backward direction of time-series, according to the display operation icon designated by the input operation of the input unit 11 among the display operation icon group 70. The display controller 16a controls to display patient information (patient name, sex, birth date, and patient ID), imaging date, and imaging time of the image P1 in the main-image display area 50 sequentially in accordance with the image P1 sequentially displayed in this manner.

When an input operation of the input unit 11 for designating the icon for setting the number of display images 93 among the icons for setting the number of display images 93 to 95 is performed with the cursor K pointed thereon, the display controller 16a controls to display one image to another for the image P1 in the main-image display area 50. When an input operation of the input unit 11 for designating the icon for setting the number of display images 94 is performed with the cursor K pointed thereon, the display controller 16a controls to display two images at once for the image P1 in the main-image display area 50. When an input operation of the input unit 11 for designating the icon for setting the number of display images 95 is performed with the cursor K pointed thereon, the display controller 16a controls to display four images at once for the image P1 in the main-image display area 50.

The display controller 16a controls the slider S to move in the forward direction or the backward direction of time-series along the time scale TS in synchronization with the changeover of images sequentially displayed in the main-image display area 50. In this case, the slider S moves to indicate, for example, the position on the time scale TS where the elapsed time since the start of imaging is shown, of the image currently displayed in the main-image display area 50.

Further, when an input operation of the input unit 11 for selectively designating a desired image for among the images sequentially displayed in the main-image display area 50 is performed with the cursor K pointed thereon, the display controller 16a controls to display a thumbnail corresponding to the selectively designated image in the subimage display area 80. Specifically, the input unit 11 inputs image-designation information which selectively designates a desired image among the images sequentially displayed in the main-image display area 50. In each case where such image-designation information selectively designating the desired image is input by the input unit 11, the display controller 16a sequentially adds a thumbnail corresponding to the image selectively designated by the image-designation information in the subimage display area. In this case, the display controller 16a controls to display thumbnails SP1 to SP5 in the subimage display area 80, as shown in FIG. 3 for example. When an input operation for selectively designating the thumbnail SP1 among the thumbnails displayed in the subimage display area 80 is performed for example, the display controller 16a controls to display the image corresponding to the selectively designated thumbnail SP1 in the main-image display area 50.

When a report in which the thumbnail displayed in the subimage display area 80 is entered is created, the display controller 16a displays a report marker indicating that the report is prepared in the neighborhood of the thumbnail. In this case, when a report is prepared for each of the thumbnails SP1 to SP5, for example, the display controller 16a controls to display report markers RM1 to RM5 in the neighborhood of the thumbnails SP1 to SP5, respectively, as shown in FIG. 3. When an input operation for selectively designating a desired report marker among the report markers displayed in the neighborhood of respective thumbnails with is performed with the cursor K pointed thereon, the display controller 16a controls to display the report corresponding to the selected report marker, so that the report can be consulted.

Next, an operation of the image file creator 16b which creates an image file of a still image or a moving image based on the image group contained in the examination file will be explained. The image file creator 16b functions as an image creator which creates a still image or a moving image of the subject, and creates an image file based on the image group contained in a desired examination file selected among the examination files stored in the examination folder 15a. In this case, the image file creator 16b creates an image file in an output mode designated with designation information which is input by the input unit 11 to the control unit 16.

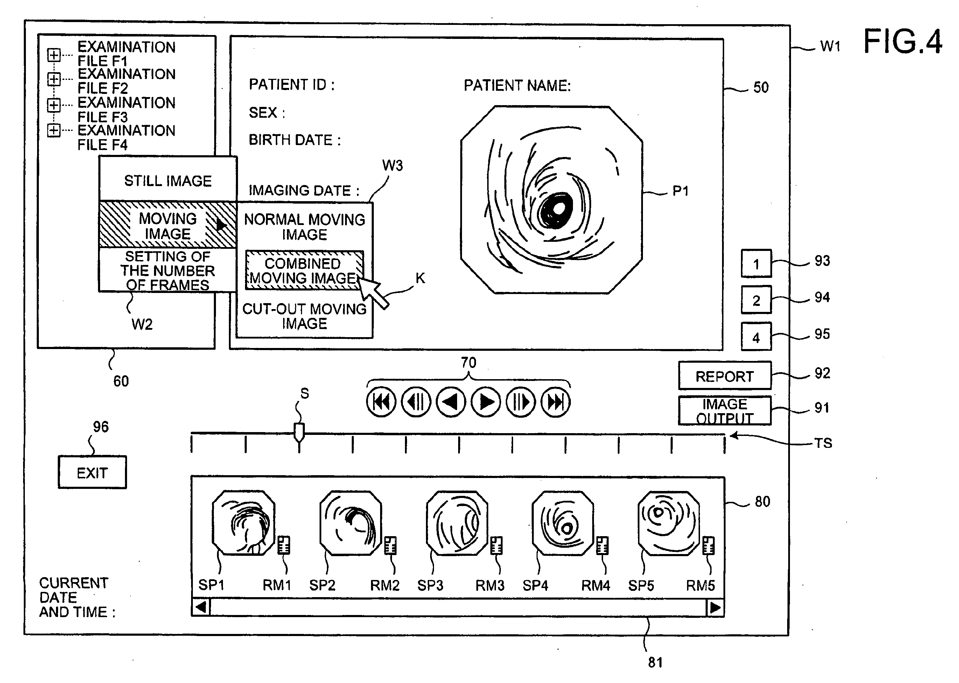

FIG. 4 is a schematic diagram exemplifying a state where a window for setting an output mode of an image file is displayed. The user performs an input operation of the input unit 11 to display a window W2 shown in FIG. 4. The window W2 displays a menu for setting an output mode of an image file to be created by the image file creator 16b. When the input unit 11 inputs designation information which selectively designates "STILL IMAGE" of the menu shown in the window W2, the image file creator 16b creates a still image file based on the image group contained in a desired examination file.

Specifically, the user uses the input unit 11 to perform an input operation for designating a desired thumbnail among thumbnails in the subimage display area 80 with the cursor K pointed thereon. In this case, the input unit 11 inputs image-designation information which designates an image of the subject corresponding to the desired thumbnail to the control unit 16. The image extracting unit 16e extracts an image designated with the image-designation information among the image group contained in the desired examination file. Accordingly, the image file creator 16b creates a still image file based on the image extracted by the image extracting unit 16e, and stores the created still image file in the management folder 15b of the storage unit 15.

The still image file created in this manner contains a still image having the same display content as the image (thumbnail) designated with the image-designation information input by the input unit 11, and has an output mode capable of outputting data in a general-purpose data format for a still image such as the GIF, JPEG, TIF, or the like.

In contrast, when the input unit 11 inputs designation information which selectively designates "MOVING IMAGE" of the menu shown in the window W2 shown in FIG. 4, the image file creator 16b creates a moving image file, i.e., one of a normal moving image file, a combined moving image file, and a cut-out moving image file. The normal moving image file contains normal moving images consisting of an image designated with the image-designation information input by the input unit 11 and a predetermined number of consecutive image frames which are in the vicinity of the designated image, and arranged along a time-series. The combined moving image file contains a combined moving image (Combined Movie) which is created by combining a plurality of normal moving images along the time-series. The cut-out moving image file contains cut-out moving images (Clipped Movie) which is created by arranging two images designated with the image-designation information input by the input unit 11 and at least one image between the two images along the time-series. Each of the normal moving image file, the combined moving image file, and the cut-out moving image file has an output mode which is capable of outputting data in a general-purpose data format for a moving image such as the WMV, MPEG, or the like.

Specifically, when the input unit 11 inputs designation information which designates "MOVING IMAGE" of the menu in the window W2, a window W3 for setting further detail of the output mode of the moving image file to be created by the image file creator 16b, as shown in FIG. 4. The user uses the input unit 11 to perform an input operation for designating one of "NORMAL MOVING IMAGE", "COMBINED MOVING IMAGE", and "CUT-OUT MOVING IMAGE" of the menu in the window W3 with the cursor K pointed thereon, and then performs an input operation for selectively designating at least one desired thumbnail among the thumbnails in the subimage display area 80. In this case, the input unit 11 inputs designation information which selectively designates one of "NORMAL MOVING IMAGE", "COMBINED MOVING IMAGE", and "CUT-OUT MOVING IMAGE" to the control unit 16. The image file creator 16b creates a moving image file containing one of the normal moving image, the combined moving image, and the cut-out moving image which is consistent with the item designated with the designation information input by the input unit 11 in the window W3, based on at least one image corresponding to at least one desired thumbnail which is selectively designated with the image-designation information input by the input unit 11 (i.e., the thumbnail selectively designated from the subimage area 80).

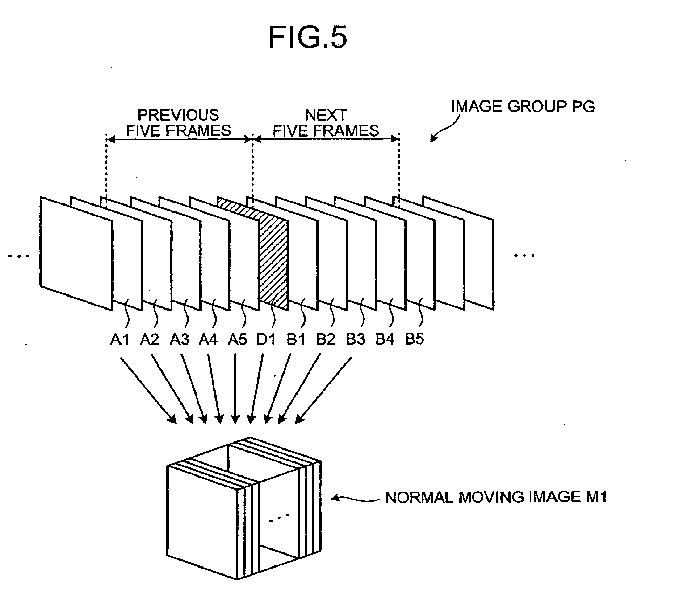

Next, an operation of the image file creator 16b which creates a moving image file containing the normal moving image, i.e., the normal moving image file will be explained in more detail. FIG. 5 is a schematic diagram for explaining the operation of creating the normal moving image file. When the input unit 11 inputs designation information which selectively designates "NORMAL MOVING IMAGE" of the menu shown in the window W3 described above, and image-designation information which selectively designates a desired thumbnail from the subimage display area 80, the image extracting unit 16e first extracts an image necessary for the process for creating the normal moving image from the image group PG contained in the desired examination file which is selected in advance by the user, as shown in FIG. 5. In this case, the image extracting unit 16e extracts an image D1 corresponding to the desired thumbnail designated with the image-designation information input by the input unit 11; a predetermined number of image frames which are consecutive in the forward direction along the time-series with respect to the image D1, i.e., five frames of images A1 to A5; and a predetermined number of image frames which are consecutive in the backward direction along the time-series with respect to the image D1, i.e., five frames of images B1 to B5.

The image D1 designated with the image-designation information is a center image arranged in a center of a partial image group (an image group containing the images A1 to A5, the image D1, and the images B1 to B5, for example) extracted from the image group PG for creating one normal moving image.

The image file creator 16b creates the normal moving image file based on the images A1 to A5, D1, and B1 to B5 extracted by the image extracting unit 16e. Specifically the image file creator 16b arranges the images A1 to A5, D1, and B1 to B5 along the time-series, and performs a compression process on the images to create a normal moving image M1 in which a moving image can be displayed by a stream play of images from the top image A1 to the end image B5. The normal moving image M1 is a partial moving image created based on the partial image group partially extracted from the image group PG contained in the desired examination file. The image file creator 16b converts the normal moving image M1 to a general-purpose data format for a moving image to create the normal moving image file. Then, the control unit 16 stores the obtained normal moving image file in the management folder 15b of the storage unit 15.

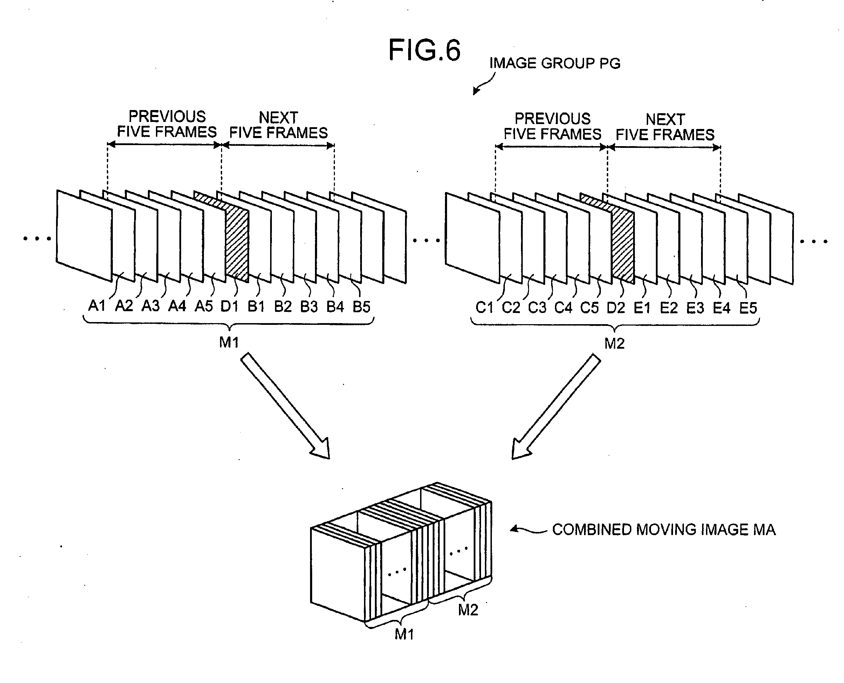

Next, an operation of the image file creator 16b which creates a moving image file containing the combined moving image, i.e., the combined moving image file, will be explained in more detail. FIG. 6 is a schematic diagram for explaining the operation of creating the combined moving image file. When the input unit 11 inputs designation information which selectively designates "COMBINED MOVING IMAGE" of the menu shown in the window W3 described above, and image-designation information which selectively designates two desired thumbnails from the subimage display area 80, the image extracting unit 16e first extracts an image necessary for the process for creating the combined moving image from the image group PG contained in the desired examination file which is selected in advance by the user, as shown in FIG. 6. In this case, the image extracting unit 16e extracts images D1 and D2 corresponding to the desired thumbnails designated with the image-designation information input by the input unit 11; a predetermined number of image frames which are consecutive in the forward direction along the time-series with respect to the images D1 and D2, i.e., five frames of images A1 to A5 and C1 to C5, respectively; and a predetermined number of image frames which are consecutive in the backward direction along the time-series with respect to the image D1 and D2, i.e., five frames of images B1 to B5 and E1 to E5, respectively.

The two images D1 and D2 designated with the image-designation information are respectively center images arranged in the centers of the two partial image groups (an image group containing the images A1 to A5, the image D1, and the images B1 to B5, and an image group containing the images C1 to C5, the image D2, and the images E1 to E5, for example) extracted from the image group PG.

As described above, the image file creator 16b arranges the images A1 to A5, D1, and B1 to B5 extracted by the image extracting unit 16e along the time-series, and performs a compression process on the images to create the normal moving image M1. Almost in the same manner as the normal moving image M1, the image file creator 16b arranges the images C1 to C5, D2, and E1 to E5 extracted by the image extracting unit 16e along the time-series, and performs the compression process on the images to create a normal moving image M2 where a moving image can be displayed by a stream play of images from the top image C1 to the end image E5. The image file creator 16b combines the end image B5 of the normal moving image M1 and the top image C1 of the normal moving image M2 by the compression process to create a combined moving image MA in which the normal moving images M1 and M2 are combined along the time-series. The image file creator 16b converts the combined moving image MA to a general-purpose data format for a moving image to create a combined moving image file. Then, the control unit 16 stores the obtained combined moving image file in the management folder 15b of the storage unit 15.

The image file creator 16b may arrange the images A1 to A5, D1, and B1 to B5 extracted by the image extracting unit 16e along the time-series, and arrange the images C1 to C5, D2, and E1 to E5 along the time-series, the image C1 being right after the image B5. Then, the compression process may be performed on the images arranged along the time-series in this way to create the combined moving image MA described above.

Here, when there is a duplication of the same image between at least two image groups extracted by the image extracting unit 16e for creating the combined moving image, the image file creator 16e deletes one of the duplicated images to create the combined moving image. For example, when the pair of images B4 and C1, and the pair of images B5 and C2 are duplicated same images respectively among the images B1 to B5 and images C1 to C5 as shown in FIG. 6, the image file creator 16b deletes one of the duplicated images B4 and C1, and one of the images B5 and C2 to create the combined moving image MA.

When the input unit 11 inputs image-designation information which selectively designates at least three desired thumbnails from the subimage display area 80, the image file creator 16b uses at least three image groups extracted by the image extracting unit 16e, i.e., at least three images (center images) respectively corresponding to at least three thumbnails designated with the image-designation information input by the input unit 11; a predetermined number of image frames (previous images) which are consecutive in the forward direction along the time-series with respect to the at least three center images, respectively; and a predetermined number of image frames (next images) which are consecutive in the backward direction along the time-series with respect to the at least three images, respectively to create the combined moving image. In this case, the created combined moving image contains at least three image groups, each consisting of the center image, the predetermined number of previous image frames, and the predetermined number of next image frames.

When the input unit 11 inputs designation information which selectively designates "SETTING OF THE NUMBER OF FRAMES" of the menu in the window W2 shown in FIG. 4, the number of extraction for each of the previous images and the next images extracted together with the center image, i.e., the predetermined number of image frames described above can be set arbitrarily. Specifically, when the input unit 11 inputs designation information which selectively designates "SETTING OF THE NUMBER OF FRAMES" in the window W2, a screen for frame setting (not shown) is displayed. Then, when the desired number of frames is input or selected via an input operation of the input unit 11, the control unit 16 sets the number for each of the previous images and the next images extracted together with the center image described above to the number of frames which is input or selected in the frame setting screen. The number of frames set in this manner is also applied to the case of creating the normal moving image described above.

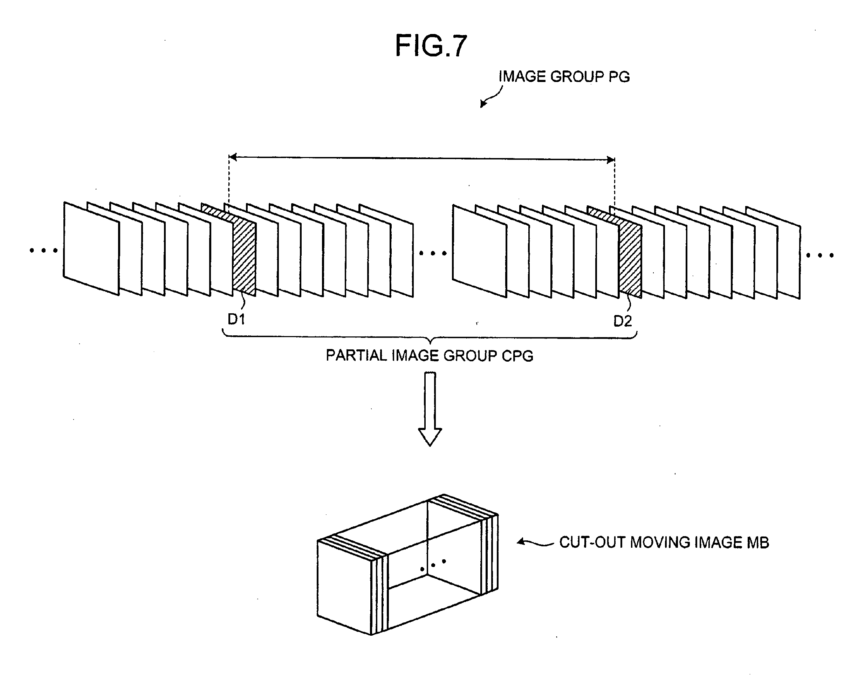

Next, an operation of the image file creator 16b which creates a moving image file containing the cut-out moving image, i.e., the cut-out moving image file will be explained in more detail. FIG. 7 is a schematic diagram for explaining the operation of creating the cut-out moving image file. When the input unit 11 inputs designation information which selectively designates "CUT-OUT MOVING IMAGE" of the menu shown in the window W3 described above, and image-designation information which selectively designates two desired thumbnails from the subimage display area 80, the image extracting unit 16e first extracts an image necessary for the process for creating the cut-out moving image from the image group PG contained in the desired examination file which is selected in advance by the user, as shown in FIG. 7. In this case, the image extracting unit 16e extracts a partial image group CPG which includes two images D1 and D2 corresponding to the desired thumbnails designated with the image-designation information input by the input unit 11; and all images between the images D1 and D2.

The two images D1 and D2 extracted in this manner are verge images arranged at both verges of the partial image group CPG, and are arranged at the top and the end along the time-series of the partial image group CPG, respectively. In other words, images included in the partial image group CPG are consecutive images along the time-series from the top image D1 to the end image D2.

The image file creator 16b arranges images included in the partial image group CPG extracted by the image extracting unit 16e along the time-series, and performs the compression process on the images to create a cut-out moving image MB in which a moving image can be displayed by a stream play of images from the top image D1 to the end image D2. The image file creator 16b converts the cut-out moving image MB to a general-purpose data format for a moving image to create the cut-out moving image file. Then, the control unit 16 stores the obtained cut-out moving image file in the management folder 15b of the storage unit 15.

Thus, the image file creator 16b can create an image file in a desired output mode designated among the still image, the normal moving image, the combined moving image, and the cut-out moving image. In this case, the image file creator 16b can easily create the still image, the normal moving image, the combined moving image, and the cut-out moving image which present an image capturing a site of interest among the image group contained in the desired examination file, for example, an image capturing a bleeding site of the subject, an affected site, or the like. When a plurality of normal moving images presenting images of a bleeding site, an affected site, or the like of the subject are created for example, the image file creator 16b can organize the plurality of normal moving images into one combined moving image. Thus, the image file creator 16b can reduce the data capacity of the moving image file (normal moving image file, combined moving image file, and cut-out moving image file) presenting images of the site of interest among the image group of the subject as much as possible, and thereby realizing a moving image file suitable for an output to the external computer and a process for playing a moving image, for example.

The image file (still image file, normal moving image file, combined moving image file, and cut-out moving image file) created by the image file creator 16b can be output in a general-purpose data format as described above. Thus, when the image file is output to the external computer for example, the image file can be easily played as a still image or a moving image by using a general-purpose application for an image display in the external computer.

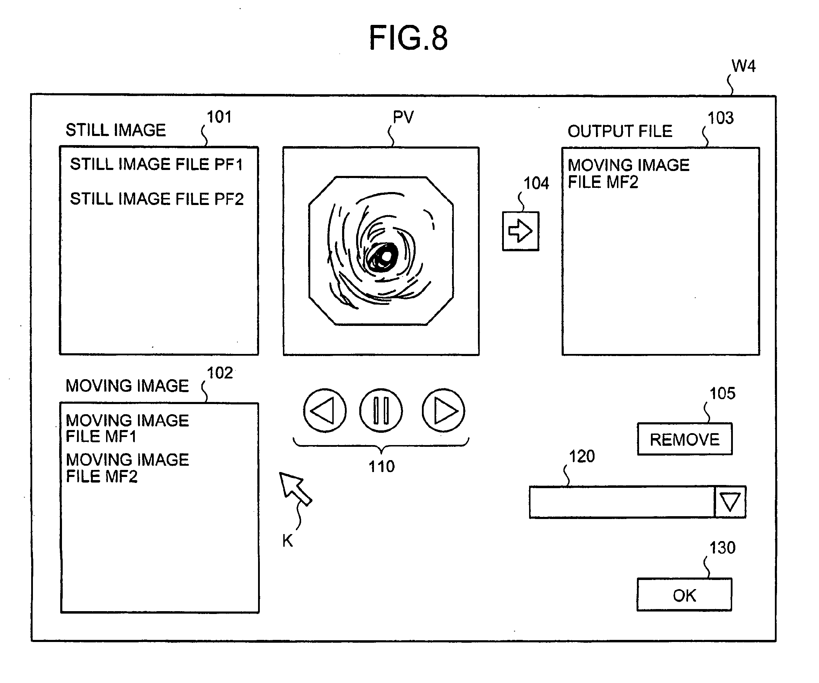

Next, an operation for outputting various kinds of image files created by the image file creator 16b to the outside will be explained. When the image file created by the image file creator 16b is output to the outside, the user first performs an input operation of clicking the image output icon 91 shown in FIG. 4 with the cursor K pointed thereon to display a predetermined window for performing an output operation and the like of the image file. FIG. 8 is a schematic diagram of one example of the window displayed on the display unit 12 for performing an output operation and the like of the image file. As shown in FIG. 8, a window W4 presents information necessary for outputting the image file created by the image file creator 16b (i.e., the still image file, the normal moving image file, the combined moving image file, and the cut-out moving image file) to the outside, and icons for performing the output operation.

Specifically, the window W4 has a still image file display area 101 which displays a list of still image files stored in the management folder 15b of the storage unit 15; a moving image file display area 102 which displays a list of moving image files stored in the management folder 15b of the storage unit 15; and an output file display area 103 which displays a list of files selected from the still image file display area 101 or the moving image file display area 102 to be output to the outside.

The window W4 also has a file designation icon 104 for displaying a desired image file selectively designated from the still image file display area 101 or the moving image file display area 102 in the output file display area 103; and a remove icon 105 for putting the image file displayed in the output file display area 103 back to the original position (the still image file display area 101 or the moving image file display area 102). The window W4 further has an output destination selecting area 120 for selecting the output destination of the image file as an output target displayed in the output file display area 103; and an OK icon for executing a file output process for outputting the image file to the output destination selected in the output destination selecting area 120.

Else, the window W4 has a preview area PV for playing the still image or the moving image contained in the image file selected from the still image file display area 101 or the moving image file display area 102; and a display operation icon group 110 for performing a display operation of the moving image played in the preview area PV, and the cursor K described above is also present in the window W4.

Here, still image files PF1 and PF2 are displayed in the still image file display area 101, and moving image files MF1 and MF2 are displayed in the moving image file display area 102, for example. In this case, the still image files PF1 and PF2 represent still image files created by the image file creator 16b and stored in the management folder 15b of the storage unit 15. The moving image files MF1 and MF2 represent moving image files, i.e., one of the normal moving image file, the combined moving image file, and the cut-out moving image file created by the image file creator 16b and stored in the management folder 15b of the storage unit 15.

The user uses the input unit 11 to perform an operation for selecting a desired still image file or a moving image file from the still image file display area 101 or the moving image file display area 102, and then performs an input operation of clicking the file designation icon 104 with the cursor K pointed thereon. In this case, the output processor 16f shifts the still image file or the moving image file selectively designated via the input operation of the input unit 11 from the management folder 15b to the input/output folder 15c. The still image file or the moving image file shifted to the input/output folder 15c is displayed in the output file display area 103.

For example, when the moving image file MF2 in the moving image file display area 102 is selectively designated, the output processor 16f shifts the moving image file MF2 from the management folder 15b to the input/output folder 15c. The moving image file MF2 shifted to the input/output folder 15c is displayed in the output ) file display area 103 as an image file of the output target. The output processor 16f sets the moving image file MF2 displayed in the output file display area 103 to be the outputting image file.

Each time when such an input operation for selectively designating a desired image file is repeatedly performed, the output processor 16f shifts the designated still image file or moving image file from the management folder 15b to the input/output folder 15c, makes the still image file and the moving image file in the input/output folder 15c displayed in the output file display area 103, and sets the still image and the moving image file to be the outputting image files.

When the user selects the remove icon 105 via an input operation of the input unit 11, the output processor 16f removes all the image files as the outputting image files displayed in the output file display area 103, and puts the image files back to the still image file display area 101 or the moving image file display area 102. In this case, the output processor 16f puts all the image files in the input/output folder 15c back to the management folder 15b.

Then, when the user uses the input unit 11 to perform input operations for selecting an output destination of the outputting image file in the output destination selecting area 120 and for selecting the OK icon 130, the output processor 16f makes copies of all still image files and moving image files displayed in the output file display area 103, i.e., copies of all image files shifted to the input/output folder 15c as the output target to create copy files respectively, and outputs all of the obtained copy files to the output destination selected in the output destination selecting area 120. The output destination to be selected in the output destination selecting area 120, for example, can be a drive for any one of FD, CD and DVD attached to the information input/output I/F 14 described above, a printer and the like to be connected via the information input/output I/F 14, or the like.

The copy file output by the output processor 16f to the information input/output I/F 14 is stored in a portable recording medium such as the FD, CD, DVD, or the like which is attached to the information input/output I/F 14 for example, and imported into the external computer via the portable recording medium. In this manner, the desired still image file or the moving image file is output to the external computer, for example. Specifically, the copy file of the desired still image file or the moving image file is output to the external computer and the like via the portable recording medium, and the original file (i.e., the still image file or the moving image file) which is the origin of the copy file is retained and managed in the management folder 15b of the storage unit 15. In this case, the lock processor 16g performs a data lock process which bans an updating process and an editing process of the data in the original file stored in the management folder 15b until the copy file output to the outside is put back to the image display apparatus 4. The data lock process for the original file performed by the lock processor 16g will be described later.

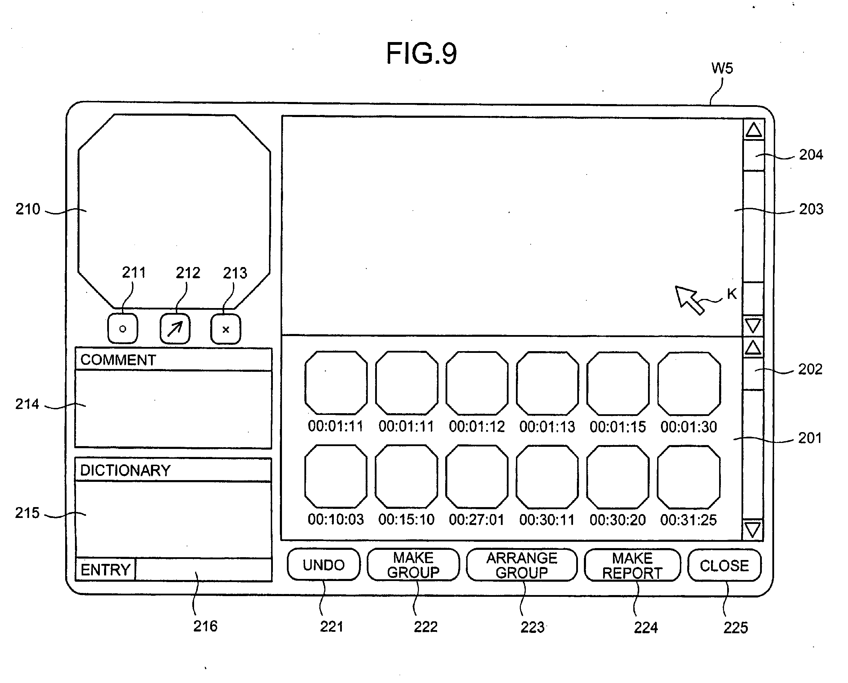

Next, an operation of the report preparing unit 16c that prepares a report in which a diagnosis result and the like of the subject are described, and an operation of the group processor 16d that separates images inserted in the report to desired groups will be explained. The user observes images of the subject 1 sequentially displayed in the main-image display area 50 to make a diagnosis on the subject 1. The user, for creating a report (medical records) in which the diagnosis result and the like of the subject 1 are described, uses the input unit 11 to perform an input operation of clicking the report icon 92 shown in FIG. 3 with the cursor K pointed thereon to make a predetermined window for creating the report appear. FIG. 9 is a schematic diagram of one example of the window displayed on the display unit for creating the report for the subject. As shown in FIG. 9, a window W5 presents information, icons, and the like which are necessary for creating the report for the subject 1.

Specifically, the window W5 has a thumbnail display area 201 which displays a thumbnail displayed in the subimage display area 80 in the window W1 described above; a scroll bar 202 for performing a scroll operation for the thumbnails displayed in the thumbnail display area 201; a grouped-image display area 203 in which the thumbnails displayed in the thumbnail display area 201 are separated into desired groups and the result thereof is displayed; and a scroll bar 204 for performing a scroll operation for images of the grouped-images (i.e., grouped-thumbnails) displayed in the grouped-image display area 203.

The window W5 also has a normal-image display area 210 which displays an image corresponding to the desired thumbnail selected from the thumbnail display area 201 or the grouped-image display area 203 (this image being similar to the image displayed in the main-image display area 50); a marker icon 211 for appending a round or oval marker to the image displayed in the normal-image display area 210; a marker icon 212 for appending an arrow marker to the image displayed in the normal-image display area 210; and a marker icon 213 for appending "X" marker to the image displayed in the normal-image display area 210. The window W5 further has a comment preparing area 214 for creating a comment to be appended to the image displayed in the normal-image display area 210; a dictionary area 215 which displays a result of the search for a desired word; and an entry area 216 which inputs a character (an initial character, for example) for searching the desired word in the dictionary area 215.

Else, the window W5 has an undo icon 221 for cancelling various kinds of processes performed in the window W5; a grouping icon 222 for separating thumbnails displayed in the thumbnail display area 201 into desired groups; a group arrangement icon 223 for changing the arrangement and the like of the grouped-images displayed in the grouped-image display area 203; a report preparation icon 224 for executing a process of preparing the report for the subject 1; and a close icon 225 for closing the window W5, and the cursor K described above is also present in the window W5.

Here, when the window W5 is opened by selecting the report icon 92 in the window W1 described above, the thumbnail selected among the images displayed in the main-image display area 50 of the window W1, and displayed in the subimage display area 80 is displayed in the thumbnail display area 201. Thumbnails in the thumbnail display area 201 are appended with elapsed times since the start of imaging, respectively as shown in FIG. 9. The user performs input operations of the input unit 11 for selecting a thumbnail in the thumbnail display area 201 and clicking the grouping icon 222 with the cursor K pointed thereon. In this case, the selected thumbnail is grouped and displayed in the grouped-image display area 203.

Then, the group processor 16d stores the setting result of the grouped-images in the storage unit 15, and achieves a group setting process of thumbnails. The grouped-images set by the group processor 16d includes at least one thumbnail.

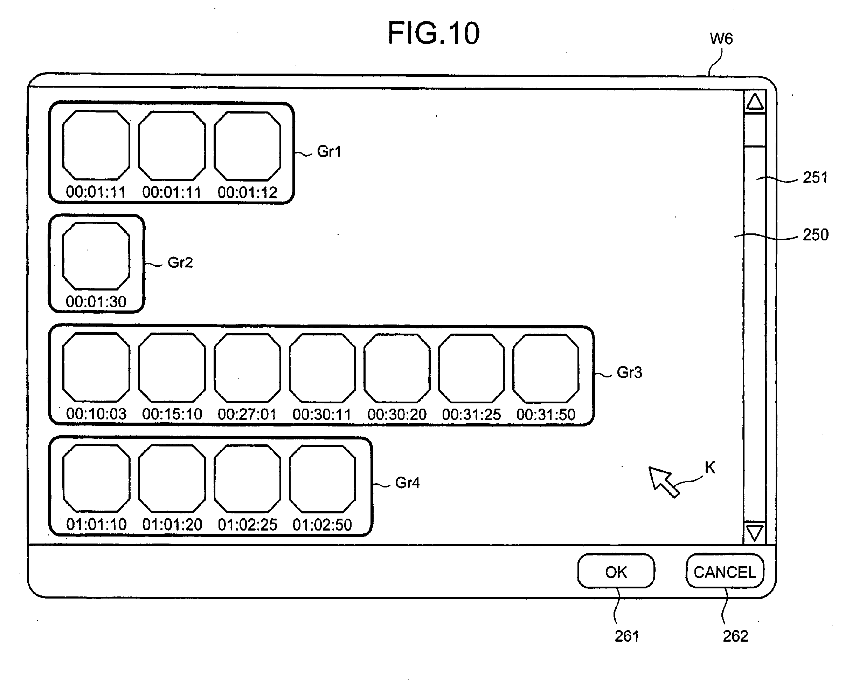

On the other hand, a window W6 is opened via an input operation for selecting the group arrangement icon 223 of the window W5 described above, grouped-images in the grouped-image display area 203 described above are displayed in a grouping area 250 in the window W6. The user uses the input unit 11 to perform an operation for changing the arrangement and the like of the grouped-images displayed in the grouping area 250. The group processor 16d changes the arrangement and the like of the grouped-images in the grouping area 250 according to the operation for changing the arrangement of the grouped-images. In this case, the group processor 16d can change the arrangement order of the grouped-images Gr1, Gr2, Gr3, and Gr4 shown in FIG. 10, for example, to the arrangement order of the grouped-images Gr1, Gr4, Gr2, and Gr3, for example. When the arrangement change operation which instructs to change the order of the thumbnails within the grouped-images is performed, the group processor 16d changes the order of the thumbnails within the grouped-images in accordance with the arrangement change operation. In this case, group processor 16d changes the order of the three thumbnails included in the grouped-images Gr1 shown in FIG. 10, for example. The grouped-images re-arranged by the group processor 16d are displayed in the grouped-image display area 203 described above.

The result of the re-arrangement process performed by the group processor 16d on the grouped-images or on the thumbnails in the grouped-images is reflected at the time of inserting the grouped-images to the report for the subject. In other words, when the report for the subject is to be prepared, the report preparing unit 16c inserts the grouped-images into the report for the subject along the arrangement order of the grouped-images which are re-arranged by the re-arrangement process by the group processor 16d.

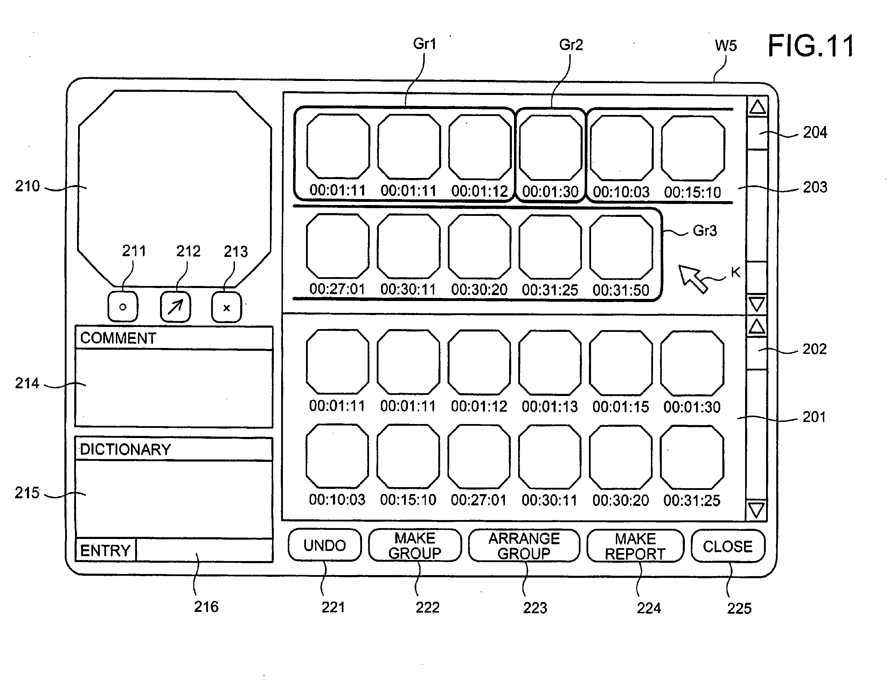

FIG. 11 is a schematic diagram exemplifying the window W5 showing a state where grouped-images are set by the group processor 16d. As shown in FIG. 11, grouped-images (grouped-images Gr1 to Gr3, for example) set by the group processor 16d are displayed in the grouped-image display area 203. In this case, the report preparing unit 16c functions to append a desired marker such as "O", "->", "X", and the like to the display content of the thumbnails included in the grouped-images, or of the thumbnails in the thumbnail display area 201.

Specifically, the report preparing unit 16c appends a desired marker such as "O", "->", "X", or the like to the image displayed in the normal-image display area 210, i.e., the image having the same display content corresponding to the thumbnail included in the grouped-images in the grouped-image display area 203 or the thumbnail in the thumbnail display area 201. More specifically, the report preparing unit 16c appends the desired marker such as the "O", "->", or "X" corresponding to the marker icons 211 to 213 to a desired position in the image displayed in the normal-image display area 210. The report preparing unit 16c stores the result of image edit in which the desired marker is appended to the image in the storage unit 15, and uses the stored result of the image edit (i.e., the image to which the desired marker is appended) when creating the report for the subject.

The report preparing unit 16c functions to append a desired comment for each grouped-images in the grouped-image display area 203 or for each thumbnail in the thumbnail display area 201. Specifically, the report preparing unit 16c associates each grouped-images selected from the grouped-image display area 203 or each thumbnail selected from the thumbnail display area 201 with the comment input in the comment preparing area 214, and stores the comment associated with each of the grouped-images or the thumbnail in the storage unit 15. The report preparing unit 16c uses the comment stored in this manner when preparing the report for the subject.

The report preparing unit 16c has a dictionary function to enable an easy operation for inputting the desired comment in the comment preparing area 214. Specifically, the report preparing unit 16c uses dictionary data stored in advance in the storage unit 15 to search at least one word whose head contains the character input in the entry area. A list of at least one word searched by the report preparing unit 16c is displayed in the dictionary area 215. When the desired word is selectively designated from the search result displayed in the dictionary area 215, the report preparing unit 16c makes the selectively designated word displayed in the comment preparing area 214. With such a dictionary function of the report preparing unit 16c, the user can perform an easy operation for inputting a desired comment in the comment preparing area 214 by using the input unit 11.

When the undo icon 221 in the window W5 is selected, the report preparing unit 16c puts the image to which the desired marker is appended correspondingly to the marker icons 211 to 213 back to the original state where no marker is appended. When the undo icon 221 in the window W5 is selected, the group processor 16d puts the grouped-images back to the original state before the grouping.

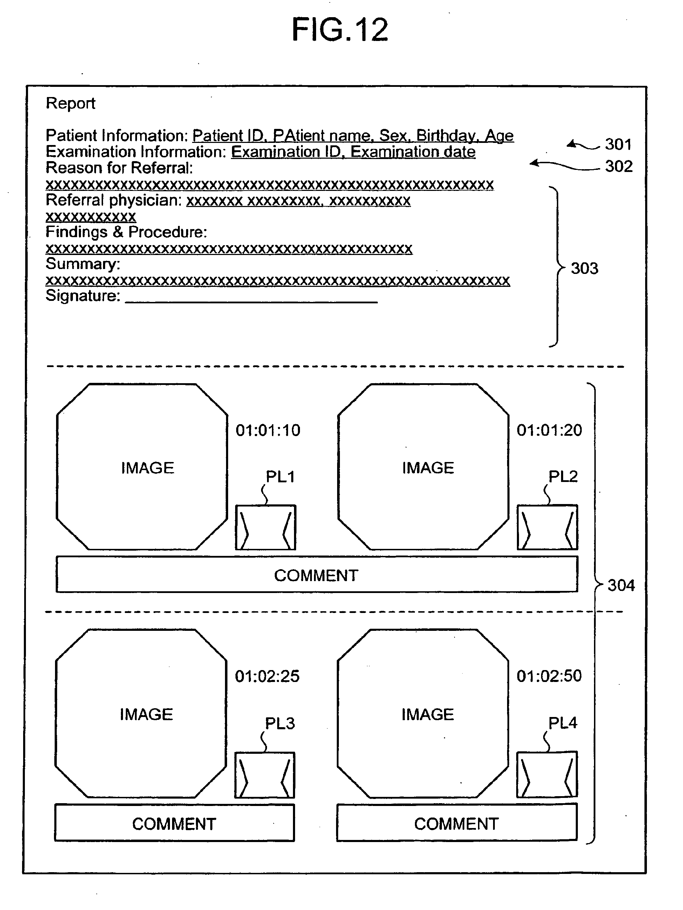

Here, the report preparing unit 16c uses, as necessary, each image having the same display content as each thumbnail in the grouped-images described above or in the thumbnail display area 201, the image to which the desired marker is appended, and the comment associated with each of such images to create a report for the subject 1. FIG. 12 is a schematic diagram of one example of the report prepared by the report preparing unit 16c. As shown in FIG. 12, the report for the subject 1 includes patient information 301 such as a patient name, sex, age, birth date, patient ID, and the like of the subject 1; examination information 302 such as a date, ID, and the like of the capsule endoscope examination on the subject 1; diagnosis information 303 such as the diagnosis result of the subject 1, the doctor name in charge of the diagnosis, a finding or summary about the diagnosis result of the subject 1, and the like; and examination result information 304 such as images of the subject 1, comment, and the like. The report for the subject 1 has a space for the signature of the doctor in charge of the diagnosis.

Specifically, the report preparing unit 16c enters the patient information 301 and the examination information 302 in the report for the subject 1 based on the patient information and the examination information contained in the examination file of the subject 1 stored in the examination folder 15a of the storage unit 15. When an instruction of concealing the patient information 301 is given via a predetermined input operation of the input unit 11, the report preparing unit 16c keeps the patient information 301 blank, or enters a marker such as "*" and the like in place of the patient information 301 to indicate that the patient information 301 is concealed.

The report preparing unit 16c enters information such as the diagnosis result of the subject 1, the doctor name in charge of the diagnosis, the finding or summary about the diagnosis result of the subject 1, and the like which are input by the input operation of the input unit 11, into a predetermined space in the diagnosis information 303.

The report preparing unit 16c further enters the image having the same display content as each thumbnail of the grouped-images described above or each thumbnail selected from the thumbnail display area 201, the image to which the desired marker is appended, the comment associated with each of such images, and the like at a predetermined position in the report as examination result information 304. In this case, the report preparing unit 16c enters the image having the same display content as the thumbnail in the grouped-images (or the thumbnail itself) in the report along the arrangement order of the grouped-images which is grouped or re-arranged by the group processor 16d. Alternatively, the report preparing unit 16c enters the image having the same display content as the thumbnail selected from the thumbnail display area 201 (or the thumbnail itself) in the report in a predetermined arrangement order. When the image to be entered is an image appended with the desired marker, the report preparing unit 16c enters the image with the desired marker appended. The report preparing unit 16c enters the comment which is input in the comment preparing area 214 described above in the neighborhood of each image entered. The comment is prepared for each of the grouped-images described above or for each of the thumbnails, and is entered in the neighborhood of the image associated therewith by the report preparing unit 16c, as shown in FIG. 12. Else, the report preparing unit 16c enters the elapsed time of the image to be entered in the report since the start of the imaging, and imaging region markers PL1 to PL4 which indicates the imaging region of the image, for each image.

In this way, the report preparing unit 16c creates the report for the subject 1 carrying the patient information 301, examination information 302, diagnosis information 303, examination result information 304, and the like, and stores a report file including the obtained report for the subject 1 in the management folder 15b of the storage unit 15. The report file is, via a predetermined input operation of the input unit 11, output to a printer and the like through the information input/output I/F, for example, in almost the same manner as the image file described above.

When the report file is output to the external computer and the like, the output processor 16f shifts the report file to the input/output folder 15c as an outputting file, creates a copy file of the report file, and outputs the copy file to the information input/output I/F 14, in almost the same manner as the case of the image data described above. The copy file output to the information input/output I/F 14 is stored in the portable recording medium such as the FD, CD, DVD, and the like attached to the information input/output I/F 14 to be imported into the external computer via the portable recording medium. In this case, the original file as an origin of the copy file, i.e., the examination file of the subject 1 is retained and managed in the management folder 15b of the storage unit 15. The lock processor 16g performs the lock process which bans an updating process and an editing process of the original file stored in the management folder 15b until the copy file is put back to the image display apparatus 4, in almost the same manner as the case of the image file described above.

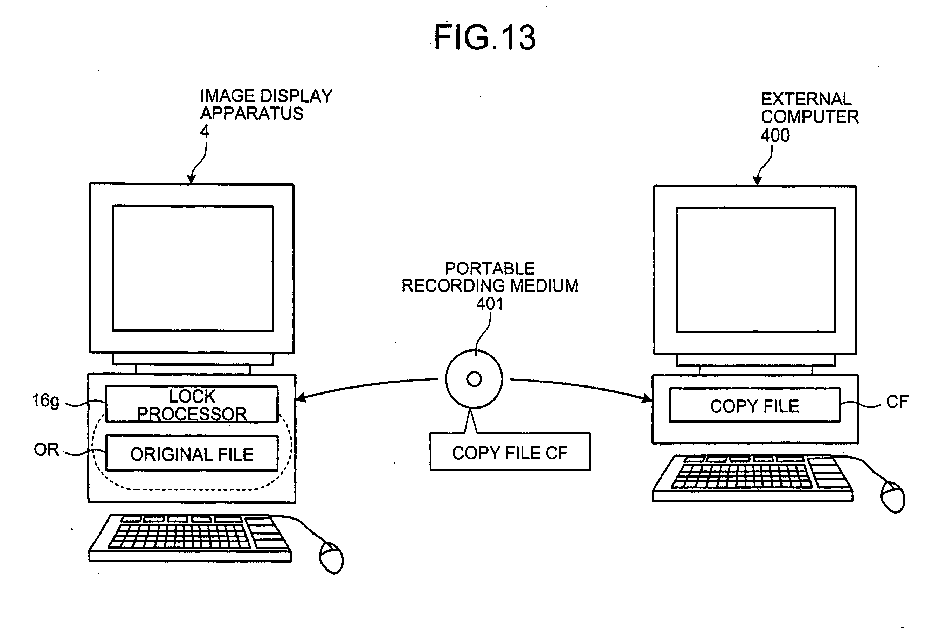

Next, an operation of the lock processor 16g which performs the data lock process with respect to the original file as an origin of the copy file output to the outside will be explained. FIG. 13 is a schematic diagram for explaining the data lock process of the original file performed by the lock processor 16g. When an image file or a report file in the desired output mode described above is output to the outside, the output processor 16f creates a copy file of the outputting image file or report file as described, and outputs the obtained copy file to the information input/output I/F 14. In this case, as shown in FIG. 13, a copy file CF is stored in a portable recording medium 401 such as the FD, CD, DVD, or the like which is attached to the information input/output I/F 14, and imported into an external computer 400 via the portable recording medium 401. Meanwhile, the output processor 16f stores an original file OR (the image file or the report file as described, for example) which is the origin of the copy file CF in the management folder 15b of the storage unit 15.

The lock processor 16g obtains a file name of the copy file CF output to the outside in this manner from the output processor 16f, and functions to perform the data lock process with respect to the original file OR specified by the same file name as the obtained file name. Specifically, when the copy file CF is output to the outside via the portable recording medium 401 for example, the lock processor 16g performs the data lock process with respect to the original file OR of the copy file CD until the copy file CF output to the outside is put back to the image display apparatus 4 to make the original file OR in a data lock state, as shown in FIG. 13. In this case, even when the user uses the input unit 11 to perform an editing operation for rewriting the data (still image, moving image, or report of the subject 1, for example) included in the original file OR, or to perform an updating operation of the data included in the original file OR, the lock processor 16g bans the updating process and the editing process of the data included in the original file OR, and protects the data against editing and updating.

While the data in the original file OR is kept protected by the lock processor 16g, the data in the copy file CF described above is edited and updated into desired data in the external computer 400. The copy file CF updated in the external computer 400 is imported into the image display apparatus 4 via the portable recording medium 401, as shown in FIG. 13. Specifically, the control unit 16 reads out the copy file CF (i.e., the copy file CF updated in the external computer 400) in the portable recording medium 401 which is attached to the information input/output I/F 14. In this case, the input processor 16h stores the copy file CF in the input/output folder 15c of the storage unit 15, and obtains the file name of the copy file CF.

Based on the file name of the copy file obtained by the input processor 16h, the lock processor 16g, checks whether the original file specified by this file name is stored in the management folder 15b or not. Specifically, when the lock processor 16g obtains the original file OR specified by the file name of the copy file CF from the management folder 15b, the lock processor 16g releases the data lock state with respect to the original file OR and permits editing process and updating process with respect to the original file OR. At the same time, the input processor 16h overwrites the original file OR with the data in the copy file CF (i.e., the data updated in the external computer 400) and obtains the original file OR including the data updated in the external computer 400. The input processor 16h stores the original file OR obtained in this manner in the management folder 15b and deletes the copy file CF after completing the updating process of the original file OR. The control unit 16 retains and manages the original file OR in the management folder 15b as a file subject to editing process and updating process, similarly to the other image file, the report file, or the like.

The copy file described above is input/output between the image display apparatus 4 and the external computer 400 via the portable recording medium 401 which can be detachably attached to both of the information input/output I/F 14 and the external computer 400. However, the present invention is not limited to this, and the copy file described above may be input/output between the image display apparatus 4 and the external computer 400 via a communication line such as a telephone, the Internet, or the like. In this case, the information input/output I/F 14 may be configured to have a communication I/F which can be connected to the communication line.

The lock processor 16g performs the data lock process with respect to the original file specified by the file name of the copy file output to the outside (the original file having the same file name as the copy file, for example). However, the present invention is not limited to this, and the data lock process may be performed with respect to the original file to which a flag and the like for specifying the copy file and the original file is appended. In this case, the output processor 16f appends a flag specifying both of the original file OR and the copy file CF to be output to the outside, outputs the copy file CF appended with the flag to the outside, and stores the original file OR appended with the flag in the management folder 15b. The lock processor 16g performs the data lock process with respect to the original file OR appended with the flag, and releases the data lock state of the original file OR when the lock processor 16g obtains the copy file CF appended with the flag.

In the embodiment of the present invention as described above, at least one image included in the plurality of images selected among the series of images of the subject which are captured by the capsule endoscope is grouped to set at least one grouped-images, or the plurality of images are separated into at least two groups to set at least two grouped-images, and then a desired comment is appended to each of the grouped-images. Thus, when there are images which are to be appended with the same comment among the plurality of images, it is not necessary to input the same finding to the plurality of images repeatedly, and possible to enable inputting the comment to each grouped-images which has grouped at least two images to be appended with the same comment. As a result, it is possible to realize an image display apparatus capable of simplifying the input operation for creating the report for the subject.

### INDUSTRIAL APPLICABILITY

As described above, the image display apparatus according to the present invention is useful for making a diagnosis on a subject based on the observation (examination) result of images capturing the alimentary canal of the subject along a time-series, specifically suitable as an image display apparatus capable of creating a report showing a diagnosis result of the subject by a simple operation.

## Claims

1. An image display apparatus (4) comprising:
a display unit (12);
a control unit (16) including a display controller (16a) configured to display a series of intra-subject images captured along a time-series on the display unit (12) in a main-image display area (50) of a first window (W1); and
an input unit (11);
wherein the display controller (16a) is configured to cause the display unit (12), by user selection of a report icon (92) in said first window (W1) displayed by the display controller, to provide in a second window (W5) a thumbnail display area (201) for displaying a plurality of thumbnails of images selectively designated in said first window (W1) by the user using the input unit, and to provide in said second window a grouped-image display area (203),
**characterized in that** the apparatus (4) further comprises:
a group processor (16d) configured to permit the user of the display apparatus, by operating the input unit, to divide a plurality of images selected from the said plurality of thumbnail images into two or more groups to obtain grouped-images (Gr1 to Gr4) wherein at least one group consists of a plurality of images, so as to enable user inputting of a comment to each of the groups; and
a report preparing unit (16c) configured to prepare a report to which the said plurality of divided images are inserted for each of the groups, wherein
the input unit (11) is configured to allow a user to input desired comments to be associated with a selected each of the groups displayed on the grouped-image display area (203), and
the report preparing unit (16c) is further configured to append to the report the desired comments for each of the associated grouped-images (Gr1 to Gr4).

2. The image display apparatus (4) according to claim 1, wherein the group processor (16d) is configured to change an arrangement order of at least two of the grouped-images (Gr1 to Gr4) arranged in a predetermined order.

3. The image display apparatus (4) according to claim 1, wherein the group processor (16d) is configured to change an arrangement order of the plurality of images arranged in each of the groups.

4. The image display apparatus (4) according to claim 1, further comprising:
a marker designating unit (K) configured to designate a marker to be appended to an image, wherein
the report preparing unit (16c) is configured to append the marker designated by the marker designating unit (K) either to an image selected among the plurality of images or to an image among the series of images corresponding to the image selected among the plurality of images.

5. The image display apparatus (4) according to claim 1, wherein the report preparing unit (16c) is configured to insert information in the report,
wherein the information inserted in the report by the report preparing unit (16c) includes patient information, examination information, diagnosis information, comment information with respect to images, an imaging region marker indicating an imaging region of the subject, and a space for the signature of the doctor who is in change of the diagnosis on the subject.

## Patentansprüche

1. Bildanzeigevorrichtung (4) mit:
einer Anzeigeeinheit (12);
einer Steuereinheit (16), die eine Anzeigesteuereinrichtung (16a) aufweist, die dazu ausgebildet ist, eine Reihe von entlang einer Zeitreihe aufgenommenen Bildern vom Körperinneren eines Patienten auf der Anzeigeeinheit (12) in einem Hauptbildanzeigebereich (50) eines ersten Fensters (W1) anzuzeigen: und
eine Eingabeeinheit (11);
wobei die Anzeigesteuereinrichtung (16a) dazu ausgebildet ist, die Anzeigeeinheit (12) dazu zu veranlassen, auf Nutzerauswahl eines Bericht-Icons (92) in dem ersten von der Anzeigesteuereinrichtung angezeigten Fenster (W1) hin, in einem zweiten Fenster (W5) einen Miniaturbildanzeigebereich (201) zum Anzeigen einer Mehrzahl von Miniaturbildanzeigen von Bildern bereitzustellen, die von dem die Eingabeeinheit nutzenden Nutzer in dem ersten Fenster (W1) selektiv gekennzeichnet werden, und in dem zweiten Fenster einen Bildergruppenanzeigebereich (203) bereitzustellen,
**dadurch gekennzeichnet, dass** die Vorrichtung (4) ferner aufweist:
einen Gruppenprozessor (16b), der dazu ausgebildet ist, es dem Nutzer der Anzeigevorrichtung zu ermöglichen, durch Bedienung der Eingabeeinheit eine Mehrzahl von Bildern, die aus der Mehrzahl von Miniaturbildern ausgewählt werden, in zwei oder mehrere Gruppen einzuteilen, um gruppierte Bilder (GR1 bis GR4) zu erhalten, wobei mindestens eine Gruppe aus einer Mehrzahl von Bildern besteht, um es dem Nutzer zu ermöglichen, einen Kommentar zu jeder der Gruppen einzugeben; und
eine Berichterstellungseinheit (16c), die dazu ausgebildet ist, einen Bericht zu erstellen, in den die Mehrzahl von eingeteilten Bildern für jede der Gruppen eingefügt werden, wobei
die Eingabeeinheit (11) dazu ausgebildet ist, es einem Nutzer zu ermöglichen, gewünschte Kommentare einzugeben, die einer ausgewählten der in dem Gruppenbildanzeigebereich (203) angezeigten Gruppen zugeordnet werden, und
die Berichterstellungseinheit (16c) ferner dazu ausgebildet ist, die gewünschten Kommentare für jedes der zugeordneten gruppierten Bilder (GR1 bis GR4) dem Report hinzuzufügen.

2. Bildanzeigevorrichtung (4) nach Anspruch 1, wobei der Gruppenprozessor (16d) dazu ausgebildet ist, eine Anordnungsreihenfolge von mindestens zwei der gruppierten Bilder (GR1 bis GR 4), die in einer vorbestimmten Reihenfolge angeordnet sind, zu ändern.

3. Bildanzeigevorrichtung (4) nach Anspruch 1, wobei der Gruppenprozessor (16b) dazu ausgebildet ist, eine Anordnungsreihenfolge der Mehrzahl von Bildern, die in jeder der Gruppen angeordnet sind, zu ändern.

4. Bildanzeigevorrichtung (4) nach Anspruch 1, die ferner aufweist:
eine Markiererzuordnungseinheit (K), die dazu ausgebildet ist, einen Markierer zuzuordnen, der einem Bild hinzuzufügen ist, wobei
die Berichterstellungseinheit (16c) dazu ausgebildet ist, den Markierer, der von der Markiererzuordnungseinheit (K) zugeordnet wird, entweder einem Bild, das aus der Mehrzahl von Bildern ausgewählt wird, oder einem Bild aus der Reihe von Bildern, die dem Bild entsprechen, das von der Mehrzahl von Bildern ausgewählt wird, hinzuzufügen.

5. Bildanzeigevorrichtung (4) nach Anspruch 1, wobei die Berichterstellungseinheit (16c) dazu ausgebildet ist, Informationen in den Bericht einzufügen,
wobei die von der Berichterstellungseinheit (16c) in den Bericht eingefügten Informationen Patienteninformationen, Untersuchungsinformationen, Diagnoseinformationen, Kommentarinformationen bezüglich Bildern, einen Bildaufnahmebereichsmarkierer, der einen Bildaufnahmebereich im Patienten anzeigt, und einen Platz für die Unterschrift des für die Diagnose des Patienten zuständigen Arztes beinhalten.

## Revendications

1. Appareil d'affichage d'images (4) comprenant :
une unité d'affichage (12) ;
une unité de commande (16) incluant un contrôleur d'affichage (16a) configuré pour afficher une série d'images intra-sujet capturées en série chronologique sur l'unité d'affichage (12) dans une zone d'affichage d'image principale (50) d'une première fenêtre (W1) ; et
une unité d'entrée (11) ;
dans lequel le contrôleur d'affichage (16a) est configuré pour amener l'unité d'affichage (12), par une sélection d'utilisateur d'une icône de rapport (92) dans ladite première fenêtre (W1) affichée par le contrôleur d'affichage, à délivrer dans une deuxième fenêtre (W5) une zone d'affichage de miniatures (201) destinée à afficher une pluralité de miniatures d'images sélectivement désignées dans ladite première fenêtre (W1) par l'utilisateur en utilisant l'unité d'entrée, et pour délivrer dans ladite deuxième fenêtre une zone d'affichage d'images groupées (203),
**caractérisé en ce que** l'appareil (4) comprend en outre :
un processeur de groupe (16d) configuré pour permettre à l'utilisateur de l'appareil d'affichage, en actionnant l'unité d'entrée, de diviser une pluralité d'images sélectionnées à partir de ladite pluralité d'images miniatures en deux groupes ou plus pour obtenir des images groupées (Gr1 à Gr4) dans lesquels au moins un groupe est composé d'une pluralité d'images, de façon à permettre à l'utilisateur de délivrer en entrée un commentaire sur chacun des groupes ; et
une unité de préparation de rapport (16c) configurée pour préparer un rapport dans lequel ladite pluralité d'images divisées est insérée pour chacun des groupes, dans lequel
l'unité d'entrée (11) est configurée pour permettre à un utilisateur de délivrer en entrée des commentaires souhaités destinés à être associés à chaque groupe sélectionné parmi les groupes affichés sur la zone d'affichage d'images groupées (203), et
l'unité de préparation de rapport (16c) est en outre configurée pour annexer au rapport les commentaires souhaités pour chacune des images groupées associées (Gr1 à Gr4).

2. Appareil d'affichage d'images (4) selon la revendication 1, dans lequel le processeur de groupe (16d) est configuré pour changer un ordre de disposition d'au moins deux des images groupées (Gr1 à Gr4) disposées dans un ordre prédéterminé.

3. Appareil d'affichage d'images (4) selon la revendication 1, dans lequel le processeur de groupe (16d) est configuré pour changer un ordre de disposition de la pluralité d'images disposées dans chacun des groupes.

4. Appareil d'affichage d'images (4) selon la revendication 1, comprenant en outre :
une unité de désignation de marqueur (K) configurée pour désigner un marqueur destiné à être annexé à une image, dans lequel
l'unité de préparation de rapport (16c) est configurée pour annexer le marqueur désigné par l'unité de désignation de marqueur (K) soit à une image sélectionnée parmi la pluralité d'images soit à une image parmi la série d'images correspondant à l'image sélectionnée parmi la pluralité d'images.

5. Appareil d'affichage d'images (4) selon la revendication 1, dans lequel l'unité de préparation de rapport (16c) est configurée pour insérer des informations dans le rapport,
dans lequel les informations insérées dans le rapport par l'unité de préparation de rapport (16c) comprennent des informations sur le patient, des informations d'examen, des informations de diagnostic, des informations de commentaires par rapport aux images, un marqueur de région d'imagerie indiquant une région d'imagerie du sujet, et un espace pour la signature du docteur qui est en charge du diagnostic sur le sujet.
